# EUROPEAN PATENT APPLICATION

(11) **EP 3 235 862 A1**
(43) Date of publication of application: **25.10.2017**
(21) Application number: 15869243.4
(22) Date of filing: 09.12.2015
(51) Int. Cl.: C08K 5/13, C07C 41/16

(54) **MULTIFUNCTIONAL SYNERGISTIC MACROMOLECULAR ANTI-OXIDATION STABILIZER AND PREPARATION METHOD AND USE THEREOF**

(30) Priority: 15.12.2014 CN 201410777531
(71) Applicant: Shaoxing Ruikang Biotechnologes Co., Inc, Shaoxing, Zhejiang 312000 (CN)
(72) Inventor: MAO, Lijuan, Brampton, Ontario L6R 0H5 (CA)
(74) Representative: Dargiewicz, Joanna
(86) International application number: PCT/CN2015/096773
(87) International publication number: WO 2016/095735

(57) **Abstract**

An antioxidative stabilizer has the following structure A, wherein R1 is a connection chain and the connection chain is a fatty chain, an aromatic structural chain or a hybrid chain of fatty and aromatic structures; R2 is B; X is O, S, N or NH or -CONR-, Z is O, S, N or NH, and X is different from Z; R is a fatty chain, an aromatic group, sterically hindered amine or sterically hindered phenol; R3 is a fatty chain, an aromatic group, sterically hindered amine or sterically hindered phenol; and R is identical to R3, or R is different from R3; and n is a positive integer including 1, n1 is a positive integer including 1, and n is identical to n1, or n is different from n1.

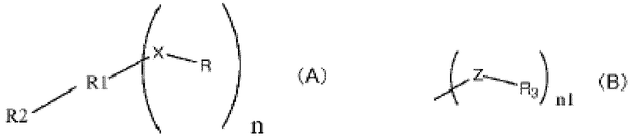

## Description

The present invention relates to a multifunctional synergistic polymer antioxidative stabilizer and a preparation method and application thereof. The multifunctional synergistic polymer antioxidative stabilizer is a new type of antioxidative stabilization additive of polymeric materials, can be directly applied to a polymer material to serve as the antioxidative stabilization additive for quality guaranteeing, color retaining, function retention and the like, and is applied to series products including plastics, rubber, fibers, coatings, paints and petroleum related products.

### BACKGROUND

The markets of antioxidative stabilizing additives around the world are very huge, and the antioxidants for single plastic materials can be consumed by about 420000 tons in the world in 2011. At present, the use quantity in the Asian-Pacific region is largest, the second largest is Europe and North America, and it is estimated that the sales amount of material antioxidative stabilizer products in the Asian-Pacific region would reach 48 billion dollars in 2016.

The demands and production of the material antioxidative stabilizing additives are transferred to emerged markets in Asia, particularly China and India, from America, Western Europe and Japan successively. At present, the growth of consumption of domestic antioxidants is faster. However, a few large international suppliers still control the world market price of the material antioxidative stabilizers.

The growth rate of the antioxidative stabilizing additive markets, particularly heat stabilizer markets, in India and the Asian-Pacific region is increased at a high speed. The demand for a special antioxidative stabilizer is increased along with application increase and technical development of polymer material. At present, special antioxidative additives and stabilizers are needed to expand the service life and application of these corresponding materials in automobile industry, organic electronics, agriculture, films, plastics, rubber, fibers, computer materials and other industries.

The demands for the antioxidative stabilizing additives are also greatly applied to development of the plastic industry, particularly the fields of vinyl polymers and PVC. PVC products are mainly used for the building field and particularly used for manufacturing pipelines and cables. More than 85% of the antioxidative stabilizers are used for this industry. It is expected that the demand in the Asian-Pacific region would further grow. The growth of the demand for light antioxidative additives will be more rapid. Particularly, the increment for polypropylene and polyethylene products will be very substantial.

Most of polymer materials need to be processed or treated at a temperature of above 200°C, but may have the problems that the service life is shortened, colors are easily destroyed, the strength is weakened, the surfaces of the materials are embrittled and fractured, etc. when continuously in a high-temperature and strong-light environment. However, the special antioxidative stabilizing additives can be introduced to prevent and reduce material damage, prolong the service life of the materials, keep beauty, ensure durability, lower the cost, reduce the yield of wastes and protect the environment.

The antioxidative stabilizing additives used in the current market are small in molecular weight and easy to volatilize, degrade or lose, and unevenly act in the materials, so that the materials are damaged to cause the problems of short service life, damaged colors, weakened strength, embrittlement, fracturing, etc. when being treated or used, particularly under the conditions of high temperature and strong light irradiation.

At the beginning of this year, articles about multifunctional antioxidative stabilizers in light-stabilization and heat-stabilization molecules report that some large international enterprises also begin to design and produce such products, and data shows that the synergistic protective effect of such intramolecular multifunctional antioxidative stabilizers on the materials is greatly better than the effect by compounding use of formulation of two kinds of antioxidants.

The polymer antioxidative stabilizer and the preparation method and the application thereof, designed and developed by the inventor, well overcome the current problems to enable the corresponding materials to keep much stable properties under the conditions of high temperature and strong light irradiation. Thus, just as the present invention appears.

### SUMMARY

In view of the above technical problems in the prior art, the purpose of the present invention is to provide a multifunctional synergistic polymer antioxidative stabilizer and a preparation method and application thereof. The multifunctionalities of novel polymer multifunctional hybrid antioxidative stabilizer was connected through a stable linker via ether bond, thioether bond, amine bond or amido bond and formed the polymer multifunctional hybrid stabillizers as a primary light-stabilizing and heat-stabilizing hybrid stabilization mode or as a bonding mode of primary and secondary antioxidative stabilizers to form the multifunctional synergistic antioxidative stabilizers by designing a suitable hybrid structure.

In order to achieve the above purpose, the present invention is realized through the following technical solution:

A multifunctional synergistic polymer antioxidative stabilizer has the following structure:

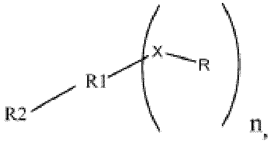

wherein R1 is a connection chain, the connection chain is a fatty chain, an aromatic structural chain or a hybrid chain of fatty and aromatic structures;

R2 is 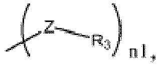
X is O, S, N or NH or -CONR-, Z is O, S, N or NH, and X is different from Z; R is a fatty chain, an aromatic group, sterically hindered amine or sterically hindered phenol, and R is identical to R3, or R is different from R3; and
n is a positive integer including 1, n1 is a positive integer including 1, and n is identical to n1, or n is different from n1.

The multifunctional synergistic polymer antioxidative stabilizer is as follows: 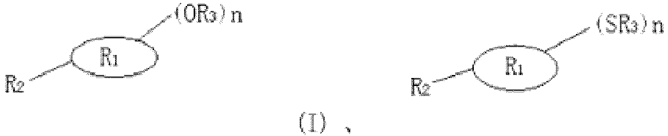 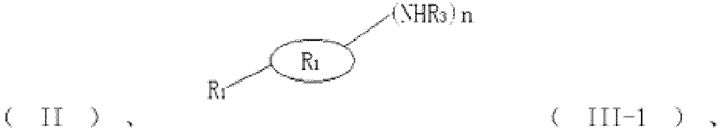 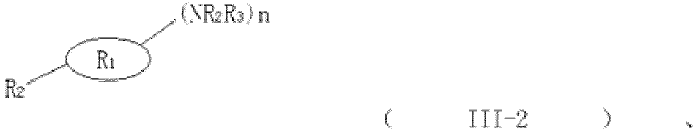 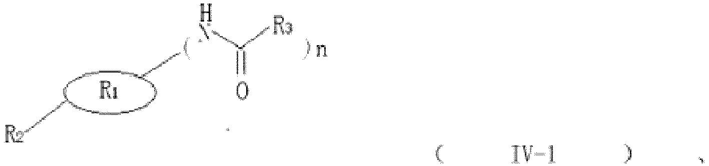 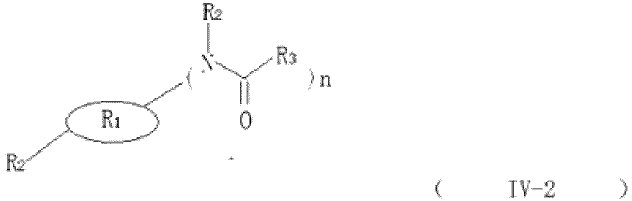 or 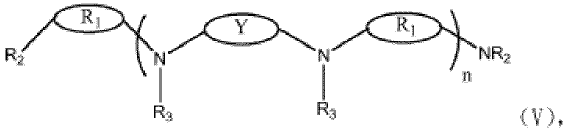 R3 is H, a fatty hydrocarbon side chain, an aromatic hydrocarbon side chain, a hybrid side chain of fatty hydrocarbon and aromatic hydrocarbon or a heteroatomic side chain; and n is a positive integer.

As an example structure of such antioxidative stabilizer, the antioxidative stabilizer is as follows: 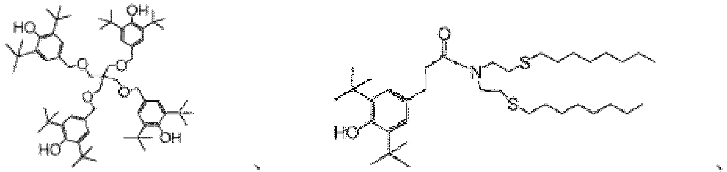 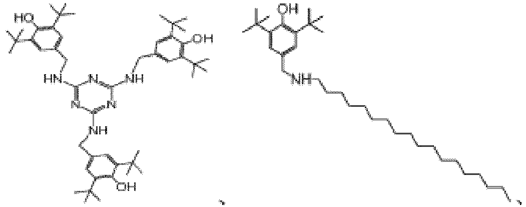 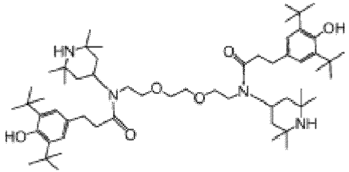 or

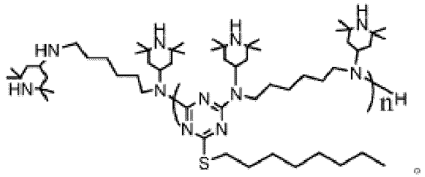

A preparation method of the multifunctional synergistic polymer antioxidative stabilizer, comprising the following steps:
wherein n is a natural number larger than or equal to 1;
adding 1 equivalent of an alcohol raw material in an anhydrous solvent of THF, DMF, acetone, ethyl acetate, toluene or acetonitrile (1; 5-15, w/v) containing 1.5-5 equivalents of NaH under nitrogen protection, stirring for 30 min- 1 h at room temperature or under the condition of heating to 30-50°C, dropwise adding 1.2-5.0 equivalents of iodide, bromide or chloride compounded with 10% of Nal or KI or activated alcohol (for example: toluene sulfate, ether trifluoroacetate and the like of alcohol), stirring a mixture at room temperature for 30 min - 1 h, heating to 40-90°C, and monitoring a reaction progress by TLC until the reaction is finished; quenching the reaction with a saturated NH₄Cl aqueous solution (1-3 times of volume of a reaction organic solvent), adding ethyl acetate or dichloromethane for fully mixing, separating an organic phase, carrying out aqueous phase extraction for three times, drying and combining with Na₂OS₄ to obtain an organic phase, filtering, removing an organic solvent under vacuum; and acquiring a solid product through recrystallization, and purifying an oily or liquid product through an extraction or silica-gel column chromatography mode, wherein a yield is 79-96%;
or
adding 1 equivalent of alcohol raw material in a solvent of THF, acetone, ethyl acetate, acetonitrile, dichloromethane, chloroform, toluene or DMF (1; 5-20, w/v) containing 1-3 equivalents of NaOH and 10-20% of tetrabutyl ammonium bromide (w/w), stirring for 1-3 h at room temperature or under the condition of heating to 30-50°C, dropwise adding 1.5-3.0 equivalents of iodide, bromide or chloride compounded with 10% of Nal or KI or activated alcohol (for example: toluene sulfate, ether trifluoroacetate and the like of alcohol), stirring a mixture at room temperature for 1 h, heating to 40-100°C for carrying out a reaction for 1-25 h, monitoring a reaction progress by TLC until the reaction is finished, acquiring a solid product through recrystallization, and purifying an oily or liquid product through an extraction mode, wherein a yield is 83-92%;
or
adding 1 equivalent of mercaptan in dry THF, DCM, acetone, acetonitrile or ethyl alcohol, methyl alcohol, chloroform, toluene or DMF solution (1; 5-15, w/v) containing 1.1-5 equivalents of iodide, bromide or chloride and compounded with 10-20% of Nal or KI or activated alcohol (for example: toluene sulfate, ether trifluoroacetate and the like of alcohol) under nitrogen protection, then adding an inorganic base such as Na₂CO₃, K₂CO₃, etc. or an organic base solution of an organic base (1.2-2.0 equivalents) such as NEt₃, DMAP, DBU, etc., stirring a mixture at room temperature for 30 min, then heating to 40-90°C, stirring for 5-12 h, monitoring a reaction progress by TLC until the reaction is finished; adding a NaCl saturated aqueous solution and equal amount of ethyl acetate or dichloromethane in a reaction system for fully mixing, separating an organic phase, washing an aqueous phase with the same organic solvent for 3 times, drying and combining with Na₂OS₄ to obtain an organic phase, filtering, and removing an organic solvent under vacuum; acquiring a solid product through recrystallization; and obtaining a liquid or oily product through a silica-gel column chromatography or extraction separation mode, wherein a yield is 78-97%;
or
adding 1 equivalent of amine in a solvent of dichloromethane, ethyl acetate, acetone, acetonitrile, THF, ethyl alcohol, methyl alcohol, chloroform, methylbenzene or DMF, (1; 5-20 w/v), dropwise adding 1-3 equivalents of organic iodide, bromide (10-50% Nal, w/w), chloride (10-50% Nal, w/w) or activated alcohol compounds while stirring under nitrogen protection, and (MsO-, TsO-, TfO-) also in a solution of an organic solvent containing 1-3 equivalents of base (an inorganic base or organic base including NaOH, KOH, Na₂CO₃, K₂CO₃, Net₃, DBU, DMAP and the like), adding 10% of tetrabutylammonium bromide according to a proportion when using the inorganic base, stirring for 1-3 h at room temperature, then heating to 40-90°C, and additionally stirring for 1-25 h; cooling, washing with a NH₄Cl aqueous solution to remove basic and water-soluble impurities, carrying out recrystallization to acquire a solid product in an organic phase, and purifying a liquid or oily product through a silica-gel column chromatography or extraction mode, wherein a yield is 67-93%;
or
dissolving or suspending 1 equivalent of amine and 1 equivalent of base (inorganic base or organic base) in an anhydrous solvent of dichloromethane, THF, MTBE, acetone, acetonitrile, chloroform, toluene or DMF (1:5-20, w/v), dropwise adding 1-2 equivalents of carbonyl chloride in the same dry solvent (1:5-10, w/v) solution at 0-10°C under nitrogen protection, stirring a mixture at 0-10°C for 30 min, carrying out reaction for 3-4 h at room temperature or under the condition of heating to 30-60°C, and monitoring a reaction progress by TLC until the reaction is finished; adding (1:20) dichloromethane, ethyl acetate or MTBE and 0.1N of hydrochloric acid iced solution, mixing, separating out an organic phase, washing an aqueous phase with the same organic solvent for 2 times, drying and combining with Na₂SO₄ to acquire an organic phase, filtering, and concentrating; acquiring a solid product through recrystallization, and carrying out silica-gel column chromatography separation or extraction to acquire a liquid or viscous oily product, wherein a yield is 75-96%;
or
controlling the average molecular weight of a multifunctional antioxidative stabilizer macromolecular product from 1000 daltons to 5000 daltons by using a multifunctional group raw material to regulate the size of n; and
dissolving 1 equivalent of diamine and polyamine into dry dichloromethane, ethyl acetate, THF, acetone, acetonitrile, ethyl acetate, methyl alcohol, chloroform, toluene or DMF (1:5-15, w/v), then dropwise adding a polyiodide, polybromide or polychloride organic raw material (adding 10-50% of Nal or KI and corresponding Bu₄NBr in bromide and chloride) while stirring under nitrogen protection, stirring at room temperature for 30 min-1 h, then heating to 40-100°C for carrying out a reaction for 6-72 h until precipitates are not increased; filtering out a solid powder product (which is white or pale yellow); and washing with a dichloromethane solvent for 3 times, (1: 2 = w/v), wherein a yield is about 87-100%.

The multifunctional group raw material may be other multifunctional group raw materials except polyamine, such as polyhydric alcohol, polymercaptan, polybasic acid, polyaldehyde, poly acyl chloride and the like.

A reaction equation of the preparation method of the multifunctional synergistic polymer antioxidative stabilizer is as follows:

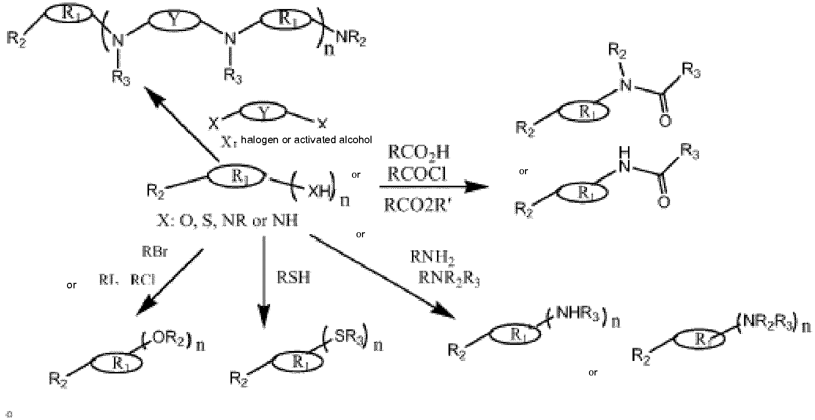

An application of the multifunctional synergistic polymer antioxidative stabilizer as an antioxidant is disclosed.

Preferably, an application of the multifunctional synergistic polymer antioxidative stabilizer in plastics, rubber, petroleum products, coatings, fiber products or paints is disclosed.

According to the present invention, with the thioether bond, a secondary amine bond or a tertiary amine bond and an ether bond as a bridge, a link spacer with a design function connects various antioxidative functional fragments including a light-stabilization functional fragment, a heat-stabilization functional fragment and a processing-stabilization functional fragment to form a new type of antioxidative stabilizer with the design function. On one hand, the polymer antioxidative stabilizer of the present invention can regulate the matching property of the antioxidative stabilizer and a macromolecule specific structure through the link spacer and a substitution side chain so as to improve the poor defects of migration, leakage and extraction of the antioxidative stabilizer in macromolecules; and on the other hand, novel antioxidative stabilizers with different hybrid multifunctional synergistic effects may be generated by introducing species of antioxidative functional groups. When the introduced functional groups are in a same type (for example, main heat-stabilization sterically hindered phenol functional fragments and main light-stabilization sterically hindered amine functional fragments), a novel heat-stabilization and light-stabilization antioxidant, having large molecular weight and good matching performance with the macromolecules, may be generated by designing link spacer fragments and the side chain structure. When the introduced antioxidative functional fragments belong to antioxidative mechanisms of different species (for example, main antioxidative functional heat-stabilization sterically hindered phenol and light-stabilization sterically hindered amine, main antioxidative functional sterically hindered phenol or sterically hindered amine fragments and auxiliary antioxidative functional fragment thioether or phosphite ester), a novel multifunctional hybrid synergistic antioxidative stabilizer may be formed. The property of the polymer hybrid synergistic antioxidative stabilizer of the present invention is not only addition of the properties of different antioxidants, but also embodies higher strength in molecule protection superiority and matching with specific macromolecules with different structures.

The present invention designs and invents a multifunctional hybrid slow-release novel antioxidative stabilizer with long time effect and useful characters of stabilizing hydrolysis, acid, base and the like. With the stable ether bond, amine bond or amido bond as the primary light-stabilization and heat-stabilization hybrid bonding mode, the multifunctional synergistic antioxidative stabilizer is formed by designing a suitable hybrid structure.

The multifunctional synergistic polymer antioxidative stabilizer of the present invention has the multifunctional hybrid synergism with stable bonding, has advantages on the characteristics of heat stabilization, hydrolysis stabilization, acid stabilization, base stabilization and the like, makes up for the disadvantages of similar products in the current market and lays a foundation of developing a new generation of effective antioxidative stabilizer.

### DETAILED DESCRIPTION

The present invention will be further described below in combination with specific embodiments. However, the protection scope of the present invention is not limited to this.

### Embodiment 1

1. Structure formula 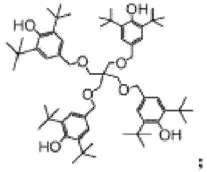
2. Synthetic route: 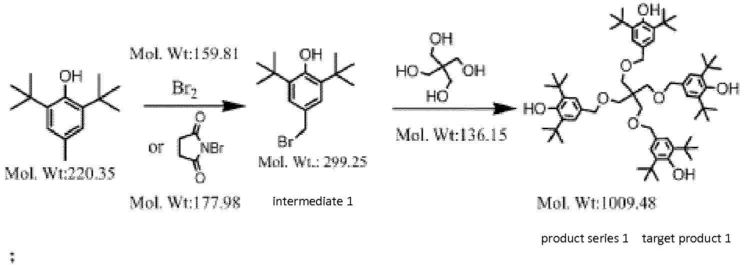
3. Synthetic step 1 of 4-bromomethyl-2, 6-di-tert-butylphenol (intermediate 1) is as follows:
   dissolving 5g of 2, 6-di-tert-butyl-4-methylphenol (22.69 mmol) into a solvent (20-50 mL) of CCl₄, CHCl₃, dichloromethane, THF, toluene, DMF and the like, dropwise adding a solvent solution (15-50 mL) of liquid bromine (above 1.2-1.5 mmol) under nitrogen protection and illumination of an ultraviolet lamp (a 350 watt mercury lamp), wherein the dropwise adding speed is determined along with the speed of the reaction; monitoring the reaction process by TLC; stirring for 5-20 min after titration is finished, removing an organic solvent under vacuum, and directly using an oily product in the next step.
4. Synthetic step 2 of 4-bromomethyl-2, 6-di-tert-butylphenol (intermediate 1) is as follows:
   dissolving 5.0g of 2, 6-di-tert-butyl-4-methylphenol (22.69 mmol) into a solvent (20-50 mL) of CCl₄, CHCl₃, dichloromethane, THF, chlorobenzene and the like, dropwise adding a solution of the same solvent (20-60 mL) of 4.1g of NBS (22.90 mmol) containing 3-10% of benzoyl peroxide; and enabling a mixture to reflux for 2-5 h, cooling to room temperature, filtering out a solid suspended matter, removing an organic solvent under vacuum, and directly using light brown liquid in the next step.
5. Preparation of a target product 1 is as follows:
   dissolving 2g of pentaerythritol (1.98 mmol), potassium hydroxide or sodium hydroxide (11.89 mmol) and tetrabutylammonium bromide (0.5 mmol) into 20 mL of THF, acetonitrile, acetone or DMF, stirring at room temperature for 1 h, heating to 50-70°C, and stirring for 30min-2h; cooling to room temperature, adding 3.49g of 2,6-di-tert-butyl-4-(bromomethyl)phenol (15.45 mmol) under nitrogen protection, enabling a mixture to reflux for 18h, monitoring the reaction progress by TLC until a raw material of pentaerythritol disappears and a main product point is formed; adding 0.1 N of iced hydrochloric acid solution and dichloromethane or ethyl acetate with the same volume, fully mixing, separating out an organic phase, extracting an aqueous phase with the same organic solvent for 2 times, drying the organic phase with anhydrous Na₂SO₄, filtering, removing the organic solvent under vacuum, and carrying out purification through silica-gel column chromatography to obtain 3.98 g of the target product 1, wherein a yield is 83.7%.
   ¹H NMR(400MHz, CHCl₃), δ(ppm): 7.26(s,CHCl₃ contained in CDCl₃), 7.15(s, 2H, 2CH), 7.00(s, 2H, 2CH), 6.92(s, 2H, 2CH), 6.87(s, 2H, 2CH), 6.66(s, 4H, 2CH₂), 5.62(s, 4H), 3.17(s, 4H, 2CH₂), 2.29(s, 4H, 2CH₂), 2.26(s, 4H, 2CH₂), 1.22-1.43(m, 72H, 24CH₃).

### Embodiment 2

1. Structure formula 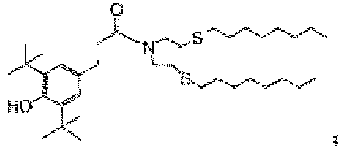
2. Synthetic route: 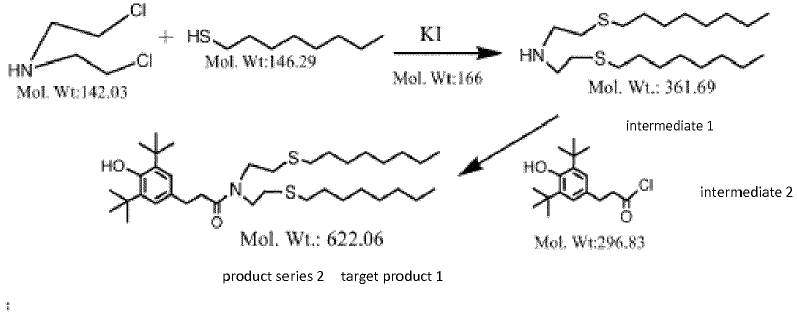
3. Preparation of the intermediate 1 is as follows:
   dropwise adding 6.1 g of mercaptan (42.30 mmol) in a solution of THF, acetone, acetonitrile, dichloromethane, ethyl alcohol and the like (1: 5-20, w/v) containing 3g of 2, 2-triethane amine (21.12 mmol) and 10-50% of KI, heating a mixture to 40-90°C, carrying out a reaction for 3-9 h, and monitoring the reaction process by TLC until the reaction is finished; removing an organic solvent under vacuum, adding an NaCl aqueous solution and the dichloromethane or ethyl acetate, fully mixing, separating out an organic phase, and extracting an aqueous phase for 2 times; and drying the organic phase with anhydrous Na₂SO₄, filtering, concentrating, and directly using the intermediate 1 in the next step.
4. Preparation of the intermediate 2 is as follows:
   dissolving 5.93 g of 3-(3, 5-di-tert-butyl-4-hydroxyphenyl) propionic acid (21.30 mmol) into dry THF, dichloromethane, ethyl acetate, MTBE or acetone (1: 5-20, w/v), cooling to 0-10°C, dropwise adding 1.82 mL of oxalyl chloride (21.50 mmol) and 0.5 mL of DMF under nitrogen protection, stirring for 30 min, rising the temperature, stirring at room temperature for 2-7 h, and monitoring the reaction process by TLC until the reaction is finished; removing an organic solvent and excessive oxalyl chloride under vacuum, and directly using residual intermediate 2 in a next reaction.
3. Preparation of a target product 1 is as follows:
   dissolving the intermediate 1 (21.12 mmol) and the intermediate 2 (21.30 mmol) into an organic solvent of dry acetone, THF, ethyl acetate, acetonitrile, toluene, dichloromethane and the like, cooling to 0-10°C, dropwise adding triethylamine (21.30mmol), stirring a mixture at 0-10°C for 1 h, then stirring at room temperature for 3-7 h, and monitoring the reaction process by TLC until the reaction is finished; and adding 0.1 N of iced hydrochloric acid solution and dichloromethane or ethyl acetate with the same volume in a reaction system, fully mixing, separating out an organic phase, washing the organic phase with 0.1N of iced hydrochloric acid for 2 times, than washing the organic phase with an NaCl saturated solution for 2 times, drying with anhydrous Na₂SO₄, filtering, removing an organic solvent under vacuum, and carrying out purification through silica-gel column chromatography to acquire 9.87g of the target product 1, wherein a yield is 75.1 %.
   ¹H NMR (400Mhz, CDCl₃), δ(ppm): 7.25(s, CHCl₃ is from CDCl₃), 6.97(s, 2H, 2CH), 2.76-2.99(m, 8H, 4CH₂), 1.52-1.59 (m, 4H, 2CH₂), 1.43 (s, 18H, 2Bu^{t}), 1.26-1.43 (m, 24H, 12CH₂), 0.88 (t, 6H, ³J_{HH} = 7.20,2CH₃).

### Embodiment 3

1. Structure formula 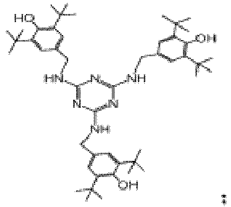
2. Synthetic route: 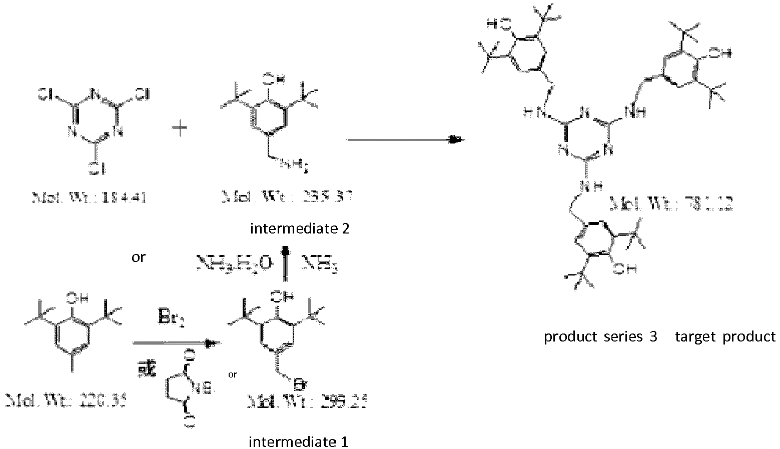
3. A synthetic step 1 of 2, 2, 6, 6-di-tert-butyl-4-bromomethyl phenol (intermediate 1) is as follows:
   dissolving 5g of 2, 6, 6-di-tert-butyl-4-methylphenol (27.11 mmol) into a solvent (1: 5-20, w/v) of CCl₄, CHCl₃, CHCl₃, CH₂Cl₂, THF, chlorobenzene, toluene or dibromoethane (1: 5-20, w/v), dropwise adding a solvent solution of bromine (28.01-37.57mmol) with the same volume under nitrogen protection and illumination of a 350 watt mercury lamp, and monitoring the reaction process by TLC; and stirring for 5-40 min after dropwise adding, removing an organic solvent under vacuum to acquire a reddish brown oily product, and directly using the oily product in the next step, wherein a yield is 95-100%.
4. A synthetic step 2 of 2, 2, 6, 6-di-tert-butyl-4-bromomethyl phenol (intermediate 2) is as follows:
   dissolving 5g of 2, 6, 6-di-tert-butyl-4-methylphenol (27.11mmol) into a solvent (1: 5-20, w/v) of CCl₄, CHCl₃, CHCl₃, CH₂Cl₂, THF, chlorobenzene, toluene or dibromoethane (1: 5-20, w/v), adding 3-10% of benzoyl peroxide, heating to refluxing, dropwise adding a solvent solution of 6.3g of NBS (35.25mmol) with the same volume, refluxing for 2-5 h after dropwise adding; and cooling to room temperature, filtering to obtain a suspended solid, concentrating a filtrate under vacuum to acquire a reddish oily product, and directly using the oily product in the next step, wherein a yield is 90-95%.
5. A synthetic step of 2, 2, 6, 6-di-tert-butyl-4-aminomethyl phenol (intermediate 2) is as follows:
   dissolving 3g of 2, 6, 6-di-tert-butyl-4-bromomethyl phenol into a solvent of THF, acetone, ethyl alcohol, methyl alcohol and the like, adding ammonia water or introducing ammonia gas, carrying out a reaction at room temperature for 2-6 h, monitoring the reaction process by TLC until a benzyl bromide raw material disappears, adding dichloromethane, petroleum ether, ethyl acetate, methylbenzene or MTBE to extract a benzyl amine product, adding the benzyl amine product in an organic phase (10mlx3); drying and combining with anhydrous Na₂SO₄ to acquire the organic phase, filtering the organic phase, removing an organic solvent of a filtrate under vacuum to acquire a faint yellow waxy solid, and directly using the faint yellow waxy solid in the next step, wherein a yield is 81-93%.
6. Preparation of a target product 1 is as follows:
   dissolving 1g of cyanuric chloride (5.42mmol) into an anhydrous solvent of dichloromethane, THF, MTBE, ethyl acetate, ethyl acetate, acetone, DMF and the like (1; 5-20, w/v), adding 5.75g of Na₂CO₃ (5.42 mmol) and the prepared crude benzyl amine (21.68 mmol) under nitrogen protection, stirring a mixture at room temperature for 1 h, heating to 40-80°C for 2-18 h, and monitoring a reaction progress by TLC until the reaction is finished; adding an NaCl aqueous solution, carrying out extraction with dichloromethane or ethyl acetate (10mlx3), drying and combining with Na₂SO₃ to acquire an organic phase, filtering, concentrating under vacuum, and carrying out silica-gel column chromatography to acquire 3.18g of white solid, wherein a yield is 75.2%. ¹H NMR(400MHz, CDCl₃), δ(ppm): 7.31 (t,3H,3CH), 7.30(s, CHCl₃ is from CDCl₃), 7.18 (t, 3H, 3CH), 2.35 (s, 6H, 3CH₂), 1.31 (s, 54H, 18CH₃).

### Embodiment 4

1. Structure formula 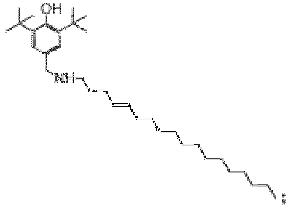
2. Synthetic route: 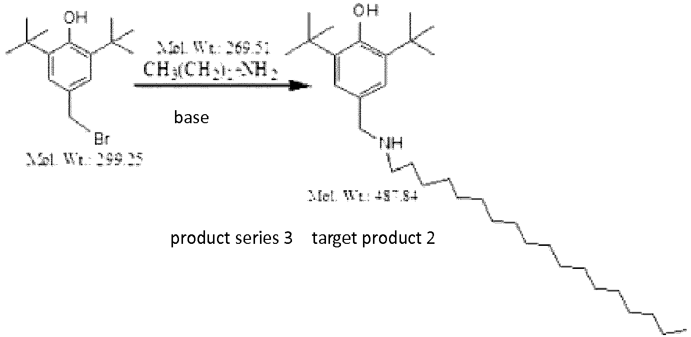
3. A preparation method of a target product 2 comprises the following steps:
   dissolving 1g of 2, 6, 6-di-tert-butyl-4-bromomethyl phenol (3.342 mmol) into a solvent (1: 5-20, w/v) of dichloromethane, ethyl acetate, petroleum ether, THF, MTBE, acetone, ethyl alcohol and the like, dropwise adding the 2, 6, 6-di-tert-butyl-4-bromomethyl phenol in a solvent, with the same volume and the same solvent, of n-octadecylamine and a base including K₂CO₃, Na₂CO₃, NaOH, triethylamine, DBU, DMAP and the like under nitrogen protection, stirring a mixture at room temperature for 1-5 h, and monitoring the reaction process by TLC until the reaction is finished; adding an NaCl aqueous solution, carrying out extraction with an organic solvent of dichloromethane, ethyl acetate, MTBE or petroleum ether to acquire a product (10mlx3), drying with anhydrous Na₂SO₄, filtering, removing an organic solvent under vacuum, and carrying out silica-gel column chromatography to acquire a yellow oily product, wherein a yield is 83-92%. ¹H MNMR(400MHz, CDCl₃), δ(ppm): 7.34 (s, CHCl₃ is from CDCl₃), 7.15 (s, 2H, 2CH), 3.45 (m, 2H, CH₂N), 2.76 (m, 2H, CH₂N),1.13-1.49 (m, 52H).

### Embodiment 5

1. Structure formula 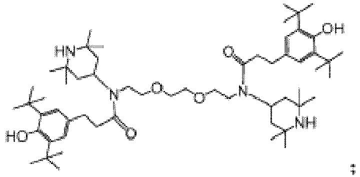
2. Synthetic route: 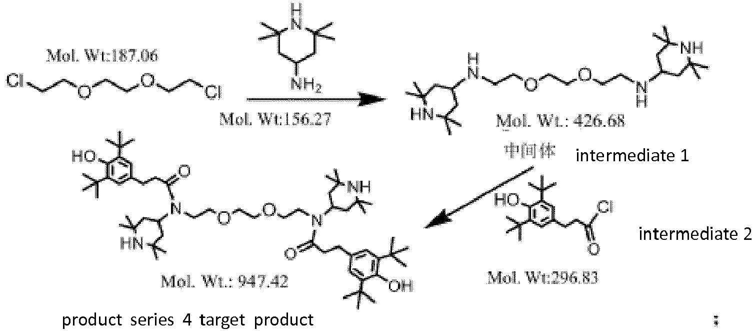
3. Synthesis of an intermediate 1 comprises the following steps:
   dissolving 6g of 1,2-dichloroethyl ethyl ether (32.08 mmol) into a solvent (1: 5-20, w/v) of acetone, acetonitrile, toluene, ethyl alcohol or MTBE, and adding 10-20% of Nal or KI, 65.00mmol of organic or inorganic base such as Na₂CO₃, K₂CO₃, NEt₃, DBU, NaOH and the like and 10.16g of 4-amino-2,2,6, 6-tetramethylpiperidine (65.00mmol) in a solution with the same volume and the same solvent; heating a mixture to 40-70°C, stirring for 3-18 h, and monitoring the reaction process by the TLC, cooling to room temperature, adding an NaCl aqueous solution, extracting the intermediate 1 (20mlx3) with dichloromethane, ethyl acetate, MTBE or petroleum ether for 3 times, drying an organic phase with anhydrous Na₂SO₄, filtering, concentrating a filtrate in vacuum, and carrying out silica-gel column chromatography to acquire a faint yellow powdery intermediate 1, wherein a yield is 81-92%.
4. A synthetic step of an intermediate 2 is as follows:
   dissolving 5.93 g of 3-(3, 5-di-tert-butyl-4-hydroxyphenyl) propionic acid (21.30 mmol) into dry THF, dichloromethane, ethyl acetate, MTBE or acetone (1: 5-20, w/v), cooling to 0-10°C, dropwise adding 1.82mL of oxalyl chloride (21.50mmol) and 0.5mL of DMF under nitrogen protection, stirring for 30min, rising the temperature, stirring at room temperature for 2-7h, and monitoring the reaction process by TLC until the reaction is finished; and removing an organic solvent and excessive oxalyl chloride under vacuum, and directly using the residual intermediate 2 in a next reaction.
5. A target product 1 is prepared by the following steps:
   dissolving 3g of the intermediate 1 (7.06mmol) and 7.36mmol of base such as Na₂CO₃, K₂CO₃, NEt₃, DMAP, DBU and the like into an anhydrous solvent of acetone, THF, dichloromethane, acetonitrile or ethyl acetate, cooling to 0-10°C, dropwise adding a solution, with the same volume and the same solvent, of 14.25mmol of the intermediate 2, stirring a mixture at 0-10°C for 30 min and at room temperature for 1-3 h after dropwise adding, heating to 40-60°C, and additionally stirring for 2-5 h; and filtering out an insoluble matter, concentrating a filtrate until removing a common solvent, adding petroleum ether with the same volume, cooling to 0°C while stirring, and collecting 4.79g of precipitated white solid, wherein a yield is 71.7%.
   ¹H NMR (400MHz,DMSO-D6), δ(ppm): 6.92 (s, 2H, 2CH), 6.89 (s, 2H, 2CH), 6.75 (s, CH₂Cl₂ solvent), 4.01 (m,4H,2OCH₂), 3.58 (sb, H₂O), 3.41 (m, 4H, 2OCH₂), 2.71 (m, 6H, 2NCH₂, 2NCH), 2.50 (m, DMSO is from DMSO-d6), 2.42 (m, 4H, 2CH₂), 2.26 (m, 4H, 2CH₂), 1.59 (m, 4H), 0.93-1.48 (m, 64H).

### Embodiment 6

1. Structure formula 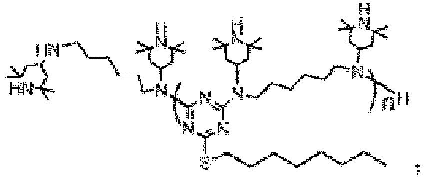
2. Synthetic route: 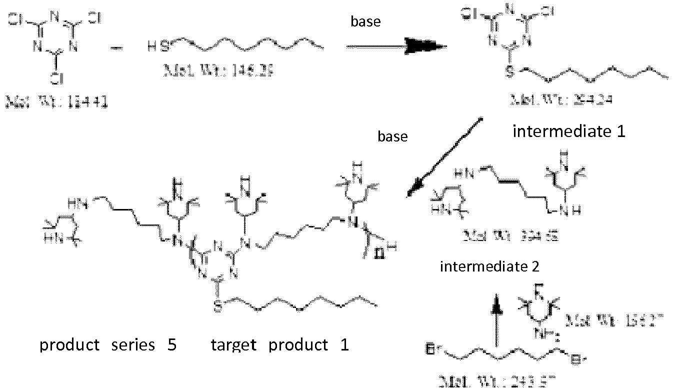 ;
3. Synthesis of an intermediate 1 comprises the following steps:
   dissolving 1g of cyanuric chloride (5.42mmol) and 5.42mmol of Na₂CO₃, K₂CO₃, NEt₃, DBU or DMAP into a solvent of dry acetone, dichloromethane, acetonitrile, THF or toluene, adding 1.03g of octanethiol (7.05mmol) under nitrogen protection, stirring a mixture at room temperature for 1 h, heating to 35-65°C for 2-7 h, and monitoring a reaction progress by TLC until the reaction is finished; and directly using the reaction in synthesis in a next synthesis.
4. Synthesis of an intermediate 2 comprises the following steps:
   dissolving 3g of 1,6-dibromohexane (12.30 mmol), 10-30% of Nal or KI and 25mmol of base such as Na₂CO₃, K₂CO₃, NEt₃, DBU, DMAP and the like into a solvent (1: 7-20, w/v) of dichloromethane, acetone, THF, MTBE, acetonitrile, toluene, ethyl alcohol and the like, and adding 3.88g of 4-amino-2,2,6, 6-tetramethylpiperidine (24.80 mmol); stirring at room temperature for 1 h, heating to 40-70°C, stirring for 3-15 h, and monitoring the reaction process until the reaction is finished; and adding an NaCl aqueous solution, extracting the intermediate 2 (15mlx3) with dichloromethane, ethyl acetate or MTBE, drying and combining with anhydrous MgSO₄ to acquire an organic phase , filtering, removing a solvent under vacuum, and directly using the intermediate 2 of a crude product in the next step.
5. Preparation of a target product 1 (high polymer) comprises the following steps:
   adding equimolar intermediate 2 in a reaction system of equimolar intermediate 1, and adding equimolar organic or inorganic base such as Na₂CO₃, K₂CO₃, NEt₃, DBU or DMAP at the same time; stirring a reaction mixture at room temperature for 1 h, then heating to 50-90°C, and stirring for 5-18 h; regulating the high molecular weight of a product through the heating temperature and the reaction time; and cooling to room temperature, adding an iced solution, washing away water-soluble impurities to acquire an oily matter which may be dissolved into an organic solvent, a waxy solid and a white solid product as the target product 1, wherein a yield is 73-91%. ¹H NMR (400Mhz, CDCl₃), δ (ppm): 7.32 (s, CHCl3 is from CDCl₃), 4.86 (m, 2H, 2CHN), 3.41 (m, 2H, 2CHN), 3.05 (m, 8H, 4CH₂N), 1.98 (m, 2H, CH₂S), 1.67 (m,8H), 1.11-1.49(m,63H).

The above embodiments are used merely to explain the inventive concepts of the present invention, not to limit protection of the right of the present invention. Immaterial changes made to the present invention by utilizing the concepts all fall into the protection scope of the present invention.

## Claims

1. A multifunctional synergistic macromolecular antioxidative stabilizer, having the following structure: 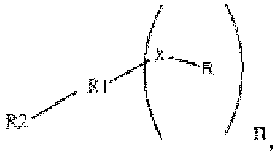 wherein R1 is a connection chain, the connection chain is a fatty chain, an aromatic structural chain or a hybrid chain of fatty and aromatic structures; R2 is 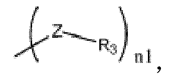 X is O S, N or NH or-CONR- Z is O, S, N or NH, and X is different from Z; R is a fatty chain, an aromatic group, sterically hindered amine or sterically hindered phenol, and R is identical to R3, or R is different from R3; n is a positive integer including 1, n1 is a positive integer including 1, and n is identical to n1, or n is different from n1.

2. The multifunctional synergistic macromolecular antioxidative stabilizer according to claim 1, wherein the antioxidative stabilizer is as follows: 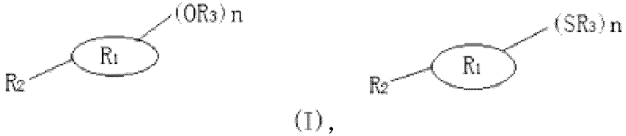 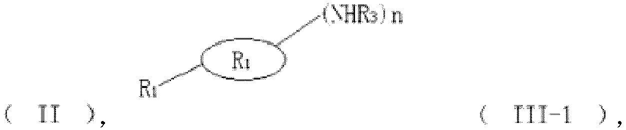 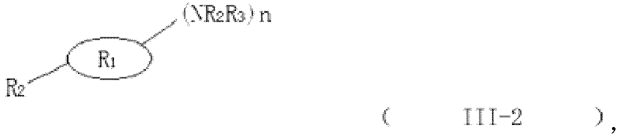 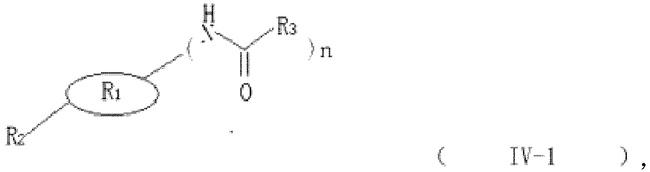 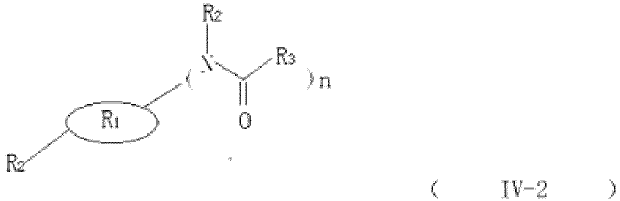 or 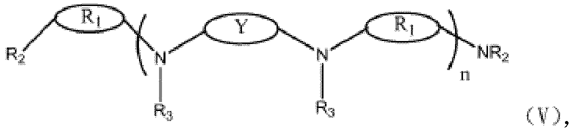 R3 is H, a fatty hydrocarbon side chain, an aromatic hydrocarbon side chain, a hybrid side chain of fatty hydrocarbon and aromatic hydrocarbon or a heteroatomic side chain; and n is a positive integer.

3. The multifunctional synergistic macromolecular antioxidative stabilizer according to claim 2, wherein the antioxidative stabilizer is as follows: 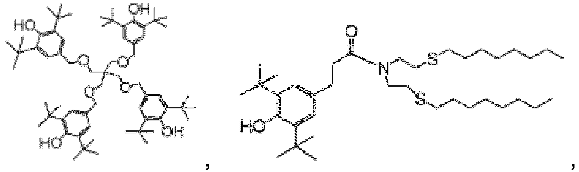 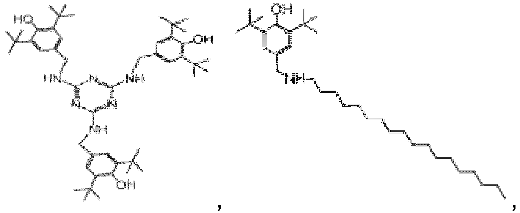 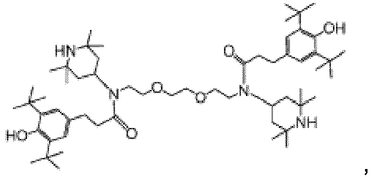 or

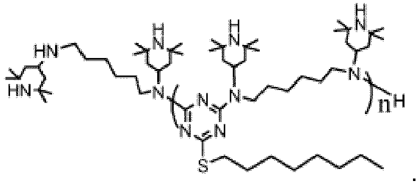

4. A preparation method of the multifunctional synergistic macromolecular antioxidative stabilizer according to claim 2, wherein n is a natural number larger than or equal to 1; comprising the following steps:
adding 1 equivalent of an alcohol raw material in an anhydrous solvent of THF, DMF, acetone, ethyl acetate, chloroform, toluene MTBE, DME or acetonitrile containing 1.5-3 equivalents of NaH or t-BuONa or t-BuOK under nitrogen protection, stirring for 10 min - 30 min at room temperature, dropwise adding 1.0-2.5 equivalents of iodide, bromide or chloride compounded with 10% Nal or KI or activated alcohol, stirring a mixture at room temperature for 30 min - 1 h, monitoring a reaction progress by TLC and heating to 40-90°Cuntil the reaction is finished; quenching the reaction with a saturated NH₄Cl aqueous solution, adding ethyl acetate or dichloromethane or petroleum ether or methylbenzene or MTBE or DME for fully mixing, separating an organic phase, carrying out aqueous phase extraction for three times, drying and combining with Na₂OS₄ to obtain an organic phase, filtering, removing an organic solvent under vacuum; and acquiring a solid product through recrystallization, and purifying an oily or liquid product through an extraction mode; or
adding 1 equivalent of alcohol raw material in a solvent of THF, acetone, ethyl acetate or acetonitrile, dichloromethane, chloroform, toluene, DME, MTBE, chlorobenzene or DMF containing 1-3 equivalents of NaOH or KOH or Bu₄OH and 10% of tetrabutyl ammonium bromide, stirring for 5 min-30 min at room temperature; dropwise adding 1.0-3.0 equivalents of iodide, bromide or chloride compounded with 5-30% Nal or KI or activated alcohol, stirring a mixture at room temperature for 1-3 h, heating to 40-100°C for carrying out a reaction for 1-25 h, monitoring a reaction progress by TLC until the reaction is finished, acquiring a solid product through recrystallization, and purifying an oily or liquid product through an extraction mode; or
adding 1 equivalent of mercaptan in dry THF, DCM, acetone, THF, DME, acetonitrile or ethyl alcohol, methyl alcohol, chloroform, toluene or DMF solution containing 1.0-3.5 equivalents of iodide, bromide or chloride and compounded with 5-30% of Nal or KI or activated alcohol under nitrogen protection; then adding Na₂CO₃ or K₂CO₃, NaOH, KOH, Bu₄OH or NEt₃ or DMAP or DBU or DIPEA or pyridine, stirring a mixture at room temperature for 30 min, then heating to 40-90°C, monitoring a reaction progress by TLC until the reaction is finished; adding a NaCl saturated aqueous solution and equal amount of ethyl acetate or dichloromethane in a reaction system for fully mixing, separating an organic phase, washing an aqueous phase with the same organic solvent for 3 times, drying and combining with Na₂OS₄ to obtain an organic phase, filtering, and removing an organic solvent under vacuum; acquiring a solid product through recrystallization; and obtaining a liquid or oily product through a silica-gel column chromatography or extraction separation mode; or
adding 1 equivalent of amine in a solvent of dichloromethane, ethyl acetate, acetone, acetonitrile, THF, ethyl alcohol, methyl alcohol, chloroform, MTBE, DME, methylbenzene or DM, dropwise adding 1-3 equivalents of organic iodide, bromide, chloride or activated alcohol compounds while stirring under nitrogen protection, and in a solution of a same organic solvent containing 1-3 equivalents of NaOH or KOH or Bu₄OH or K₂CO₃ or Na₂CO₃ or DIPEA or NEt₃ or pyridine; adding 5-30% of tetrabutylammonium bromide according to a proportion when using the inorganic base, enabling the mixture to react for 1-3 h at room temperature, then heating to 40-90°C, and additionally stirring for 1-25 h; cooling, washing with a NH₄Cl aqueous solution to remove basic and water-soluble impurities, carrying out recrystallization to acquire a solid product in an organic phase, and purifying a liquid or oily product through a silica-gel column chromatography or extraction mode; or
dissolving or suspending 1 equivalent of amine and 1 equivalent of base in an anhydrous solvent of dichloromethane, THF, MTBE, DME, acetone, acetonitrile, chloroform, toluene or DMF, dropwise adding 1 equivalent of carbonyl chloride in the same dry solvent solution at 0-10°C under nitrogen protection, stirring a mixture at 0-10°C for 30 min-2 h, carrying out reaction for 3-24 h at room temperature or under the condition of heating to 30-60°C, and monitoring a reaction progress by TLC until the reaction is finished; adding dichloromethane, ethyl acetate, methylbenzene, DME or MTBE and 0.1 N of hydrochloric acid iced solution, mixing, separating out an organic phase, washing an aqueous phase with the same organic solvent for 2 times, drying and combining with Na₂SO₄ to acquire an organic phase, filtering, and concentrating; acquiring a solid product through recrystallization, and carrying out silica-gel column chromatography separation or extraction to acquire a liquid or viscous oily product; or
controlling the average molecular weight of a macromolecular product from 1000 daltons to 5000 daltons by regulating the size of n; dissolving 1 equivalent of diamine and polyamine into dry dichloromethane, ethyl acetate, THF, acetone, acetonitrile, ethyl acetate, methyl alcohol, chloroform, toluene or DMF, then dropwise adding a polyiodide, polybromide or polychloride organic matter under nitrogen protection while stirring under nitrogen protection, stirring at room temperature for 30 min, then heating to 40-100°C for carrying out a reaction for 6-72 h; controlling the average molecular weight of the polymer product through reaction temperature and time; filtering out a solid powder product; and washing with a dichloromethane solvent for 3 times to obtain a solid product, or purifying through an extraction node to acquire a viscous oily product.

5. The preparation method of the multifunctional synergistic macromolecular antioxidative stabilizer according to claim 4, wherein a reaction equation is as follows:

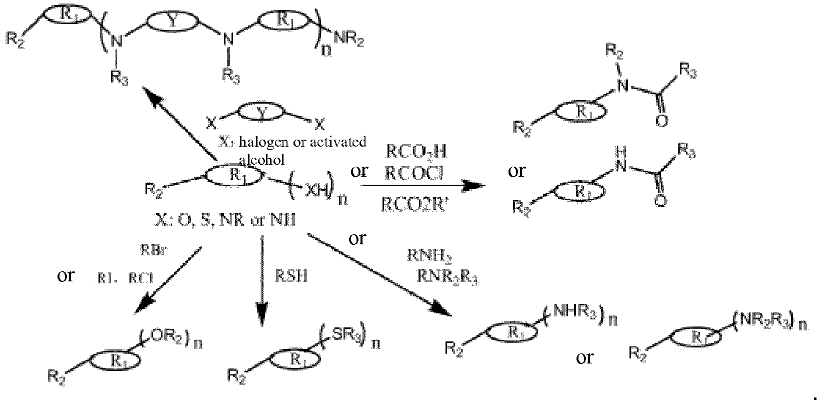

6. Use of the multifunctional synergistic macromolecular antioxidative stabilizer according to any one of claims 1-3 as an antioxidant.

7. Use of the multifunctional synergistic macromolecular antioxidative stabilizer according to claim 6 in plastics, rubber, petroleum products, coatings, fiber products or paints.
